# EUROPÄISCHE PATENTANMELDUNG

(11) **EP 2 987 528 A1**
(43) Veröffentlichungstag der Anmeldung: **24.02.2016**
(21) Anmeldenummer: 15168077.4
(22) Anmeldetag: 19.05.2015
(51) Int. Cl.: A61N 1/05, A61N 1/375, A61B 5/00, A61N 1/378

(54) **MEDIZINISCHES IMPLANTAT**

(30) Priorität: 19.08.2014 US 201462038847 P
(71) Anmelder: BIOTRONIK SE & Co. KG, 12359 Berlin (DE)
(72) Erfinder: Dörr, Thomas, 12437 Berlin (DE); Diebold, Michael, 12169 Berlin (DE)
(74) Vertreter: Galander, Marcus

(57) **Zusammenfassung**

Die Erfindung betrifft ein medizinisches Implantat (100) zum Einführen in den menschlichen und/oder tierischen Körper, mit einem Implantatkörper (102). Der Implantatkörper (102) wenigstens einen ersten und wenigstens einen zweiten Kontaktabschnitt (130, 140) aufweist, wobei die ersten und zweiten Kontaktabschnitte (130, 140) dazu vorgesehen sind, zwei eine Relativbewegung gegeneinander ausführende Gewebebereiche (210, 220) zu kontaktieren, wobei die ersten und die zweiten Kontaktabschnitte (130, 140) relativ zueinander bewegbar sind und eine Relativbewegung der Kontaktabschnitte (130, 140) in ein elektrisches Signal umwandelbar ist.

## Beschreibung

Die Erfindung betrifft ein medizinisches Implantat zum Einführen in den menschlichen und/oder tierischen Körper.

Medizinische Implantate zum Einführen in den menschlichen oder tierischen Körper sind bekannt. Es ist ebenfalls bekannt, dass derartige Implantate eine autarke Energieversorgung aufweisen können, die Energie aus dem Körper bezieht, in den diese Implantate eingesetzt wurden. Dies ist auch unter dem Begriff "energy harvesting" bekannt. Der Wirkungsgrad ist gering und die gewonnene Energiemenge daher häufig unzureichend. Dies gilt vor allem dann, wenn Implantate mit therapeutischer Energieabgabe betrachtet werden, welche einen erhöhten Energiebedarf haben.

Der Erfindung liegt die Aufgabe zugrunde, ein elektronisches Implantat zu schaffen, das einen erhöhten Wirkungsgrad aufweist.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein medizinisches Implantat zum Einführen in den menschlichen und/oder tierischen Körper vorgeschlagen, mit einem Implantatkörper, wobei der Implantatkörper wenigstens einen ersten und wenigstens einen zweiten Kontaktabschnitt aufweist. Die Kontaktabschnitte sind dazu vorgesehen, zwei eine Relativbewegung gegeneinander ausführende Gewebebereiche zu kontaktieren, wobei die ersten und die zweiten Kontaktabschnitte relativ zueinander bewegbar sind und eine Relativbewegung der Kontaktabschnitte in ein elektrisches Signal umwandelbar ist.

Vorteilhaft ist eine autonome Energieversorgung elektronischer Implantate möglich, die im bestimmungsgemäßen Einsatz zwischen zwei Körpergewebsabschnitten befestigt sind, die eine mehr oder weniger zyklische Relativbewegung zueinander ausüben. Die Erfindung kann für alle aktiven Implantate eingesetzt werden, die zwischen Körpergeweben implantiert werden, die eine wiederholte, insbesondere regelmäßige, Relativbewegung zueinander aufweisen. Die Bewegungsenergie des Gewebes kann über die damit verbundenen Kontaktabschnitte in elektrische Energie transformiert werden. Es ist eine sehr kompakte Bauweise und gute Ankopplung der Gewebebewegung möglich, wodurch eine Verbesserung des Wirkungsgrades erreicht werden kann. Die Erfindung ist besonders geeignet für dauerimplantierbare elektronische Implantate zur Diagnostik und/oder Therapie.

Gemäß einer günstigen Ausgestaltung kann der erste und/oder der zweite Kontaktabschnitt direkt oder indirekt mit einem elektrischen Generator im oder am Implantatkörper gekoppelt sein. Eine direkte Kopplung entspricht einer mechanischen Verbindung mit einem beweglichen Teil eines Generators, etwa einer Spule oder eines mit der Spule in elektromagnetischer Wirkverbindung stehenden Magneten.

Gemäß einer günstigen Ausgestaltung kann der erste Kontaktabschnitt ein Fixierelement zum Befestigen des Implantatkörpers an einem der Gewebebereiche umfassen. Das Fixierelement kann beispielsweise eine Fixierhelix sein. Zusätzlich oder alternativ kann eine Verklebung zwischen Implantatgehäuse und Gewebebereich vorgesehen sein.

Gemäß einer günstigen Ausgestaltung können der erste und/oder zweite Kontaktabschnitt mechanisch mit einem bewegbaren Element des Generators verbunden sein. So kann der Kontaktbereich mit der Spule eines Generators oder mit dem Magneten des Generators verbunden sein. Eine Bewegung des Kontaktabschnitts induziert über die elektromagnetische Wechselwirkung zwischen Spule und Magneten eine elektrische Spannung.

Gemäß einer günstigen Ausgestaltung kann der Implantatkörper mehrteilig ausgebildet sein, wobei wenigstens zwei Teile gegeneinander bewegbar sein können und jedem der Teile des Implantatkörpers ein Kontaktabschnitt zugeordnet ist. Insbesondere können die Teile mittels wenigstens eines piezoelektrischen Elements gekoppelt sein. Alternativ oder zusätzlich kann zwischen Teilen des Implantatkörpers wenigstens bereichsweise ein mechanischer Schwingungsgenerator angeordnet sein. Durch die Relativbewegung des Gewebes werden über die Kontaktabschnitte die Teile des Implantatkörpers gegeneinander bewegt, wodurch die kinetische Energie der Gewebebewegung in den Implantatkörper abgeleitet und umgesetzt werden kann.

Gemäß einer günstigen Ausgestaltung kann der Generator ein Elektrostatikgenerator sein, der über mechanische Wellen antreibbar ist. Vorteilhaft ist ein mikromechanischer Elektrostatikgenerator einsetzbar. Vorteilhaft kann zwischen den Teilen des mehrteiligen Implantatkörpers eine mechanische Struktur dergestalt vorgesehen sein, dass durch die Gewebebewegung mechanische Vibrationen erzeigt werden, welche in Frequenz uns Schwingungsform auf den Elektrostatikgenerator abgestimmt sind, so dass dieser möglichst in Resonanznähe betrieben werden kann.

Gemäß einer günstigen Ausgestaltung kann die zweite Kontaktvorrichtung ein Fixierelement zum Befestigen des Implantatkörpers an einem der Gewebebereiche sein. Damit ist eine besonders effektive Einkopplung der Relativbewegung der Gewebebereiche in das Implantat möglich.

Gemäß einer günstigen Ausgestaltung kann ein Energiespeicher im Implantatkörper vorgesehen sein. Der Energiespeicher kann ein Akkumulator sein, ein Kondensator oder ein mechanischer Schwungradspeicher. Auf diese Weise kann erzeugte elektrische Energie gespeichert werden und zur Diagnostik und/oder zur therapeutischen Energieabgabe vom Implantat in das Gewebe gezielt eingesetzt werden. Der mechanische Schwungradspeicher ist kann für kurzzeitige Energiespeicherungen genutzt werden, wobei eine Umwandlung der Bewegungsenergie in eine Rotationsenergie erfolgt und erst bei Bedarf diese Rotationsenergie in eine elektrische Energie umgewandelt wird (z.B. durch elektrisches Belasten eines Generators).

Gemäß einer günstigen Ausgestaltung kann eine Steuer- und/oder Regeleinheit im Implantatkörper vorgesehen oder mit diesem gekoppelt oder koppelbar sein, um eine therapeutische Energieabgabe gezielt zu bewirken. Eine solche Steuer- und/oder Regeleinheit ist vorzugsweise eine elektronische Schaltung, die in Abhängigkeit des therapeutischen Bedarfs die entsprechende Therapie einleitet oder unterdrückt oder variiert, wie zum Beispiel ein Zeitgeber in einem Herzschrittmacher.

Gemäß einer günstigen Ausgestaltung kann das Implantat als epikardialer Schrittmacher ausgestaltet sein. Ein solcher Schrittmacher wird zwischen Myokard und Perikard eingesetzt und kann die starken Relativbewegungen zwischen Myokard und Epikard effektiv zur Energiegewinnung nutzen.

Gemäß einer weiteren Ausgestaltung kann das Implantat in anderen Gewebebereichen, die eine regelmäßige Bewegung erfahren, wie zum Beispiel Lunge - Zwerchfell, Muskelgewebeübergänge, Wirbel- und Knochenübergänge angeordnet sein.

Günstigerweise erlaubt die Erfindung, den Wirkungsgrad der Energiegewinnung in elektronischen Implantaten durch Umwandlung mechanischer Körperbewegungen in elektrische Energie deutlich gegenüber dem bisherigen Stand der Technik zu steigern.

Die Erfindung ist nachfolgend beispielhaft, anhand von in Zeichnungen dargestellten Ausführungsbeispielen, näher erläutert. Es zeigen in schematischer Darstellung:
- Fig. 1: zeigt zur Veranschaulichung vorhandener Relativbewegungen zwischen Myokard und Perikard mit einem epikardialen Implantat mit einem Gewebebereich in einer ersten Position;
- Fig. 2: zeigt zur Veranschaulichung vorhandener Relativbewegungen zwischen Myokard und Perikard mit einem epikardialen Implantat mit einem Gewebebereich in einer zweiten Position verglichen mit Figur 1;
- Fig. 3: zeigt einen Schnitt durch ein Implantationsgebiet mit einem epikardialen Implantat nach einem Ausführungsbeispiel der Erfindung, das mit einem ersten Kontaktabschnitt in Form einer Fixierhelix in einem ersten Gewebebereich fixiert ist und mit einem zweiten Kontaktabschnitt an einem weiteren Gewebebereich fixiert ist;
- Fig. 4: zeigt einen Schnitt durch ein Implantationsgebiet mit einem zweigeteilten epikardialen Implantat in einer ersten Position nach einem Ausführungsbeispiel der Erfindung, wobei ein erster Teil des Implantats mit einem ersten Kontaktabschnitt in Form einer Fixierhelix in einem ersten Gewebebereich fixiert ist und ein zweiter Teil des Implantats mit einem zweiten Kontaktabschnitt an einem weiteren Gewebebereich fixiert ist, wobei die Implantatteile mit piezoelektrischen Elementen verbunden sind;
- Fig. 5: zeigt das Implantat aus Figur 4 in ausgelenkter Position;
- Fig. 6: zeigt einen Schnitt durch ein Implantationsgebiet mit einem zweigeteilten epikardialen Implantat in einer ersten Position nach einem Ausführungsbeispiel der Erfindung, wobei ein erster Teil des Implantats mit einem ersten Kontaktabschnitt in Form einer Fixierhelix in einem ersten Gewebebereich fixiert ist und ein zweiter Teil des Implantats mit einem zweiten Kontaktabschnitt an einem weiteren Gewebebereich fixiert ist, wobei zwischen den Implantatteilen ein mechanischer Schwingungserzeuger angeordnet ist; und
- Fig. 7: zeigt das Implantat aus Figur 6 in ausgelenkter Position.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Die Erfindung ist anhand von epikardialen Schrittmachern beschrieben. Grundsätzlich sind jedoch auch andere dauerimplantierbare elektronische Implantate zur Diagnostik und/oder Therapie geeignet, welche zwischen Körpergewebebereiche eingeführt werden, die eine Relativbewegung gegeneinander ausführen.

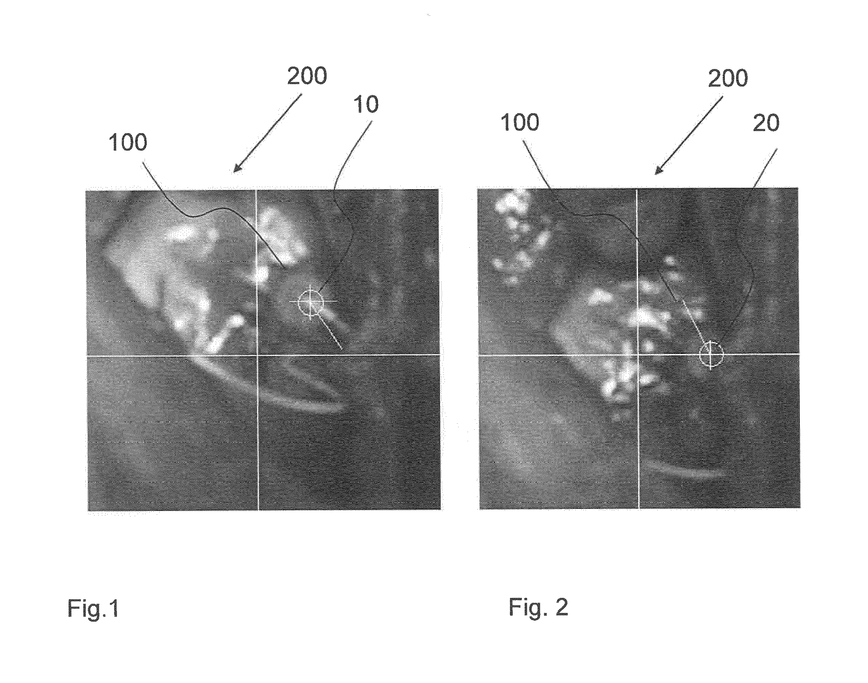

Zur Veranschaulichung der Erfindung sind in den Figuren 1 und 2 vorhandene Relativbewegungen zwischen zwei Gewebebereichen von Myokard und Perikard in einem Implantationsgebiet 200 dargestellt, in dem ein epikardiales Implantat 100 eingesetzt ist. Figur 1 zeigt das Implantationsgebiet 200 mit einem Gewebebereich in einer ersten Position 10. Figur 2 zeigt den Gewebebereich in einer zweiten Position 20. Die Relativbewegung des Gewebes zwischen den beiden Positionen 10, 20 kann zur Erzeugung elektrischer Energie im Implantat 100 ausgenutzt werden. Die pro Herzschlag von der ersten Position 10 gegenüber der am weitesten entfernten Position 20 zurückgelegte Strecke beträgt hier etwa 10 mm. Diese Bewegung wird durch eine Muskelkraft verursacht, die für die hinreichende Energiegewinnung nun entsprechend abgeleitet werden kann.

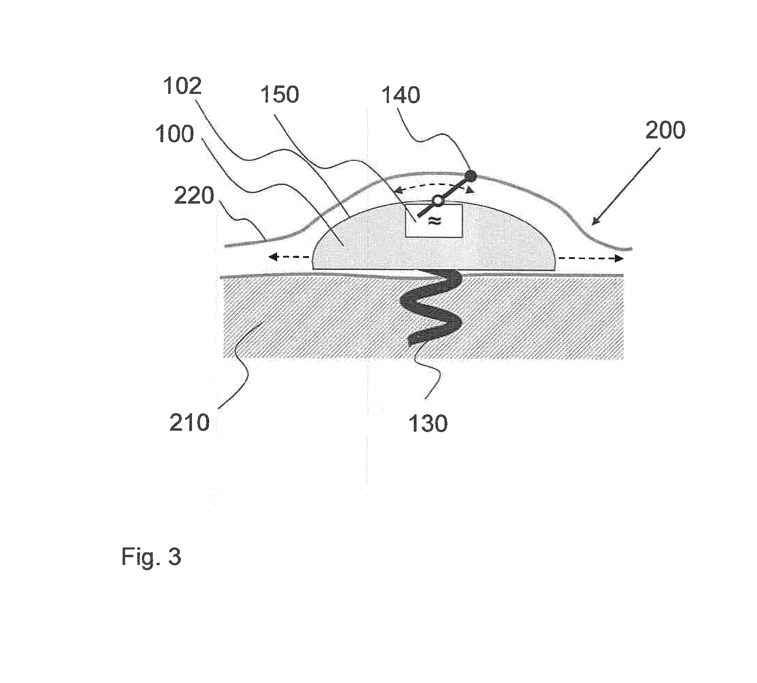

Fig. 3 zeigt einen Schnitt durch ein Implantationsgebiet 200 mit einem epikardialen Implantat 100 nach einem Ausführungsbeispiel der Erfindung. Das Implantat 100 weist ein Implantatgehäuse 102 auf, welches mit einem ersten Kontaktabschnitt 130 in Form einer Fixierhelix in einem ersten Gewebebereich 210, beispielsweise dem Myokard, fixiert ist und mit einem zweiten Kontaktabschnitt 140 an einem gegenüberliegenden weiteren Gewebebereich 220, beispielsweise dem Perikard, fixiert ist. Der zweite Kontaktabschnitt 140 ist direkt mit einem elektrischen Generator 150 im Implantatkörper 102 gekoppelt und bewegt einen Magneten des Generators 150 gegenüber einer Spule (nicht dargestellt), wenn die Gewebebereiche 210, 220 eine Relativbewegung zueinander ausführen. Der zweite Kontaktabschnitt 140 ist beweglich gelagert und kann eine Kippbewegung um ein Drehgelenk ausführen.

In diesem Beispiel ist im Implantationsgebiet 200 eine laterale Relativbewegung zwischen den beiden Gewebebereichen 210, 220 vorgegeben, die durch nach rechts und links weisende strichlierte Pfeile in der Figur angedeutet ist. Die beiden Kontaktabschnitte 130, 140 sind mit ihrem jeweiligen Gewebebereich 210, 220 verbunden und werden von diesen mitgenommen, so dass der zweite Kontaktabschnitt 140 den Magneten des Generators 150 über eine mechanische Kopplung mitnimmt, wenn der Kontaktabschnitt 140 seine Kippbewegung ausführt. Der Kontaktabschnitt 140 kann z.B. mit dem Gewebebereich 220 verklebt sein, etwa mittels eines Fibrinklebers oder eines anderen geeigneten Klebers.

Der Magnet steht in elektromagnetischer Wechselwirkung mit der Spule und induziert dort in bekannter Weise eine elektrische Spannung, welche zum Betrieb des Implantats 100, etwa zur Diagnoseausgabe, eingesetzt werden kann oder alternativ oder zusätzlich in einem nicht dargestellten elektrischen Speicher gespeichert und bei Bedarf abgerufen werden kann. Grundsätzlich kann selbstverständlich auch die Spule gegenüber dem Magneten bewegt werden.

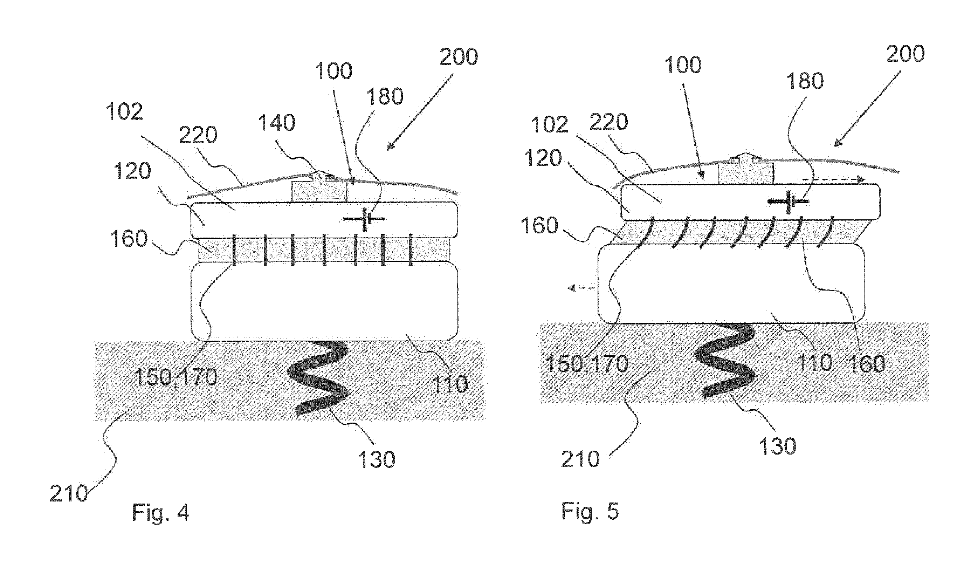

Die Figuren 4 und 5 zeigen einen Schnitt durch ein Implantationsgebiet 200 mit einem epikardialen Implantat 100 nach einem Ausführungsbeispiel der Erfindung. In diesem Ausführungsbeispiel ist das Implantat zweigeteilt. Figur 4 zeigt das epikardiale Implantat 100 in einer ersten Position, Figur 5 zeigt das Implantat 100 aus Figur 4 in ausgelenkter Position.

Ein erster Teil 110 des Implantats 100 ist mit seinem ersten Kontaktabschnitt 130 in Form einer Fixierhelix in einem ersten Gewebebereich 210, beispielsweise dem Myokard, fixiert, und ein zweiter Teil 120 des Implantats 100 ist mit seinem zweiten Kontaktabschnitt 140 an einem weiteren Gewebebereich 220, beispielsweise dem Perikard, fixiert. Die Teile 110, 120 sind über eine elastische Masse 160 verbunden, in die streifenförmige piezoelektrische Elemente 170 eingebettet sind, welche einen Generator bilden. Der zweite Kontaktabschnitt 140 ist beispielsweise als Widerhaken im zweiten Gewebebereich 220 fixiert.

Führen die Gewebebereiche 210, 220 eine laterale Relativbewegung zueinander aus, wie mit nach rechts und links weisenden strichlierten Pfeilen in Figur 5 angedeutet ist, werden die piezoelektrischen Elemente 170 verformt und induzieren eine elektrische Spannung, die als Spannungsquelle für eine Stromausgabe dienen und/oder zur Energiespeicherung mit einem Energiespeicher 180, etwa einem elektrochemischen Energiespeicher, gekoppelt sein kann.

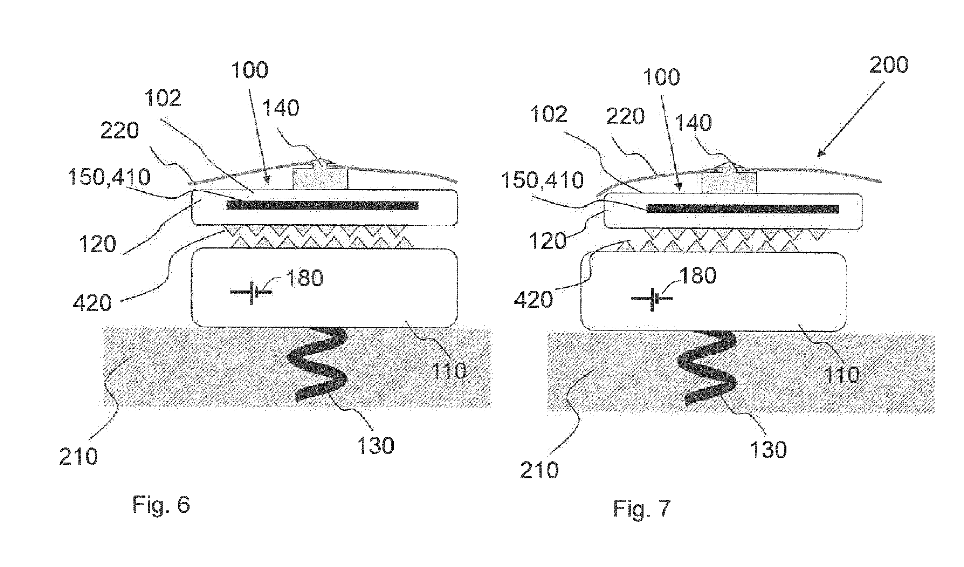

Die Figuren 6 und 7 zeigen einen Schnitt durch ein Implantationsgebiet 200 mit einem epikardialen Implantat 100 nach einem Ausführungsbeispiel der Erfindung. In diesem Ausführungsbeispiel ist das Implantat zweigeteilt. Figur 6 zeigt das epikardiale Implantat 100 in einer ersten Position, und Figur 7 zeigt das Implantat 100 aus Figur 6 in ausgelenkter Position.

Ein erster Teil 110 des Implantats 100 ist mit einem ersten Kontaktabschnitt 130 in Form einer Fixierhelix in einem ersten Gewebebereich 210, beispielsweise dem Myokard, fixiert, und ein zweiter Teil 120 des Implantats 100 ist mit einem zweiten Kontaktabschnitt 140 in Form eines Widerhakens an einem weiteren Gewebebereich 220, beispielsweise dem Perikard, fixiert. Im zweiten Implantatteil 120 ist ein Generator 150 in Form eines mikromechanischen Elektrostatikgenerators 410 angeordnet. Solche Elektrostatikgeneratoren sind allgemein auch als MEMS-Resonatoren bekannt, die über Vibrationsenergie angeregt werden können. Heutige MEMS-Resonatoren können an ihrer aktiven Chipfläche bei geeigneter Anregung derzeit etwa 150 µW/cm² erzeugen, was zu einer Versorgung eines Schrittmachersystems hinreichend ist. Diese so genannten "Highperformance electrostatic MEMS vibration energy harvester" erzeugen ihre maximale Leistung bei passender mechanischer Anregung, Frequenz und Pulsform. Vorteilhaft wird hier zwischen erstem und zweitem Teil 110, 120 des Implantats 100 eine mechanischer Schwingungsgenerator 420, z.B. eine Reibfläche, implementiert, der bei der zu erwartenden Relativbewegung einen passende Anregungsfrequenz im Teil 120 mit dem mikromechanischen Elektrostatikgenerator 410 erzeugt.

Optional kann der Generator 150, 410 zusätzlich auf einem entsprechend abgestimmten Resonator angeordnet sein (nicht dargestellt).

Optional kann in allen Ausführungsbeispielen eine Steuer- und/oder Regeleinheit (zeichnerisch nicht dargestellt) im Implantatkörper 102 vorgesehen oder mit diesem gekoppelt oder koppelbar sein, um eine therapeutische Energieabgabe zu bewirken, wozu Energie eingesetzt werden kann, die in einem entsprechenden Energiespeicher 180 im Implantat 100 gespeichert ist. Eine solche Steuer- und/oder Regeleinheit ist vorzugsweise eine elektronische Schaltung, die in Abhängigkeit des therapeutischen Bedarfs die entsprechende Therapie einleitet oder unterdrückt oder variiert, wie zum Beispiel ein Zeitgeber in einem Herzschrittmacher.

## Patentansprüche

1. Medizinisches Implantat (100) zum Einführen in den menschlichen und/oder tierischen Körper, mit einem Implantatkörper (102), **dadurch gekennzeichnet, dass** der Implantatkörper (102) wenigstens einen ersten und wenigstens einen zweiten Kontaktabschnitt (130, 140,) aufweist, wobei die ersten und zweiten Kontaktabschnitte (130, 140) dazu vorgesehen sind, zwei eine Relativbewegung gegeneinander ausführende Gewebebereiche (210, 220) zu kontaktieren, wobei die ersten und die zweiten Kontaktabschnitte (130, 140) relativ zueinander bewegbar sind und eine Relativbewegung der Kontaktabschnitte (130, 140) in ein elektrisches Signal umwandelbar ist.

2. Implantat nach Anspruch 1, **dadurch gekennzeichnet, dass** der erste und/oder der zweite Kontaktabschnitt (130, 140) direkt oder indirekt mit einem elektrischen Generator (150, 410) im oder am Implantatkörper (102) gekoppelt ist.

3. Implantat nach Anspruch 1 oder 2, **dadurch gekennzeichnet, dass** der erste Kontaktabschnitt (130) ein Fixierelement zum Befestigen des Implantatkörpers (102) an einem der Gewebebereiche (210) umfasst.

4. Implantat nach einem der vorangegangenen Ansprüche, **dadurch gekennzeichnet, dass** der erste und/oder zweite Kontaktabschnitt (130, 140) mechanisch mit einem bewegbaren Element des Generators (150) verbunden ist.

5. Implantat nach einem der vorangegangenen Ansprüche, **dadurch gekennzeichnet, dass** der Implantatkörper (102) mehrteilig ausgebildet ist, wobei wenigstens zwei Teile (110, 120) gegeneinander bewegbar sind und jedem der Teile (110, 120) des Implantatkörpers (102) ein Kontaktabschnitt (130, 140) zugeordnet ist.

6. Implantat nach Anspruch 5, **dadurch gekennzeichnet, dass** die Teile (110, 120) mittels wenigstens eines piezoelektrischen Elements (170) gekoppelt sind.

7. Implantat nach Anspruch 5, **dadurch gekennzeichnet, dass** zwischen Teilen (110, 120) des Implantatkörpers (102) wenigstens bereichsweise ein mechanischer Schwingungsgenerator (420) angeordnet ist.

8. Implantat nach einem Ansprüche 2 und 5 bis 7, **dadurch gekennzeichnet, dass** der Generator (410) ein Elektrostatikgenerator ist, der über mechanische Wellen antreibbar ist.

9. Implantat nach einem der Ansprüche 5 bis 8, **dadurch gekennzeichnet, dass** die zweite Kontaktvorrichtung (140) ein Fixierelement zum Befestigen des Implantatkörpers (102) an einem der Gewebebereiche (210) ist.

10. Implantat nach einem der vorangegangenen Ansprüche, **dadurch gekennzeichnet, dass** ein Energiespeicher (180) im Implantatkörper (102) vorgesehen ist.

11. Implantat nach einem der vorangegangenen Ansprüche, **dadurch gekennzeichnet, dass** eine Steuer- und/oder Regeleinheit im Implantatkörper (102) vorgesehen oder mit diesem gekoppelt oder koppelbar ist, um eine therapeutische Energieabgabe gezielt zu bewirken.

12. Implantat nach einem der vorangegangenen Ansprüche, **gekennzeichnet durch** eine Ausgestaltung als epikardialer Schrittmacher.
